# EUROPEAN PATENT SPECIFICATION

(11) **EP 0 609 247 B1**
(45) Date of publication and mention of the grant of the patent: **09.08.2000**
(21) Application number: 92920184.6
(22) Date of filing: 11.09.1992
(51) Int. Cl.: B08B 7/00, A61K 7/48

(54) **SKIN CLEANSER**
HAUTREINIGER
NETTOYANT EPIDERMIQUE

(30) Priority: 19.09.1991 US 762692
(43) Date of publication of application: 10.08.1994
(73) Proprietor: SMITHKLINE BEECHAM CORPORATION, Philadelphia Pennsylvania 19101 (US)
(72) Inventor: GANGADHARAN, Balgopal, Caldwell, NJ 07006 (US); HAYWARD, Marshall, A., Bridgewater, NJ 07054 (US)
(74) Representative: White, Susan Mary
(86) International application number: US9207695
(87) International publication number: WO9305893

(56) References cited:
- EP-A- 0 140 325
- FR-A- 1 587 321
- US-A- 3 645 904
- US-A- 3 708 435
- US-A- 4 126 142
- US-A- 4 508 634
- US-A- 4 586 962
- US-A- 4 743 442
- US-A- 5 019 174
- US-A- 5 139 770
- US-A- 5 143 071
- SOAP COSMETICS CHEMICAL SPECIALITIES, vol.58, no.1, 1982, NY USA page 90 'formulation ideas " aloe mask "'
- DATABASE WPI Week 8305, Derwent Publications Ltd., London, GB; AN 83-11210K & JP-A-57 207 648 (MITSUBISHI CHEM IND KK) 20 December 1982
- A.WADE, P.J.WELLER: 'Handbook of Pharmaceutical Excipients', THE PHARMACEUTICAL PRESS, LONDON * page 392 *
- J.D.ROBERTS: 'Basic Principles of Organic Chemistry', W.A.BENJAMIN, INC., NEW YORK * page 378 - page 379 *

## Description

This invention relates to a polymer-based cleanser for superficial and deep cleansing of skin.

### Area of the Invention

Skin cleaning and treating materials in the form of pastes, viscous suspensions, creams, gels and even plasticized polymeric have long been used, no doubt starting in pre-historic times. Early historical records and anecdotes describe the use of plant and animal derived pastes, gels and appliques which were applied to the skin for medicinal and cosmetic purposes. More recent history reveals a continued interest in topically applied suspensions, gels, pastes and the like which are left on the skin for a discrete period, then peeled or scrubbed off, thereby removing surface and embedded grime and debris.

EPA 0 140 325 discloses a film forming cosmetic composition comprising a copolymer of vinyl alcohol and alkyl vinyl ether in defined molar ratio ranges. This co-polymeric composition is described as being useful in a variety of cosmetic applications, but preferably in the form of an aerosol pack.

JP-A-57207 648 discloses a rapidly dryable polymer composition useful in a cosmetic pack and in different types of cleaners e.g. kitchen and record cleaners. The compositions comprise a combination of polymers, specifically a polyvinyl alcohol with a hydroxyalkylcellulose polymer.

An article in Soap Cosmetics Chemical Specialties, Vol. 58, number 1, 1982, NY, USA discloses a formulation for an aloe peel-off mask. This composition comprises a mixture of two polyvinyl alcohols of varying molecular weight and viscosity; the PVA polymers being present in the composition in an amount 35% weight/weight each, totalling 70% weight of polymer.

US Patent 4 126 142 discloses a two-stage skin treating technique wherein a film of a polystyrene sulfonate salt solution is applied to pre-determined areas of the skin and allowed to dry. Example 1 of the specification states that the face is washed to remove the dried film and is followed immediately with application of an oil formulation so as to maintain the skin in a plumped state. Compositions are disclosed comprising a film forming agent, namely a polystyrene sulfonate salt, a liquid modifier such as a plasticizer or surfactant and a viscolizer selected from, amongst others, vinyl polymers.

Hair follicles secrete a horny material throughout out the life of the individual. Normally this material is extruded and removed by washing, shaving or other cleansing processes. Age-related skin changes may interfere with this extrusion process. Follicles may dilate and fill up with horny material which can trap small velus hairs. This accumulated debris distends the follicle resulting in what are commonly called clogged pores,comedones and blackheads. Bacteria such as *P. acnes*, fungi (*P. ovale*) and a mite (*Demodex folliculorum*) contribute to follicular debris build-up. Diseases such as acne, keratosis pilaris, ichthyosis and fungal infections involve abnormal and excessive keratinazation.

It would be desirable to provide an applique which would remove surface and embedded debris without sacrificing skin comfort and which avoids redness, especially that often associated with removing plasticized appliques. This invention provides such an applique. When applied to the skin, these cleansers will adsorb surface sebum, deposited grime and soils and will attach to follicular deposits. Removing the applique removes these topical and embedded soils, grime and debris.

### Summary of the Invention

This invention covers a composition for forming an applique for cleaning or treating skin comprising a lower alcohol or alcohol/water solvent in which is dissolved between 0.1 to 20% by weight/volume of a polymer consisting essentially of a non-acrylate, pre-polymerized high molecular weight, dermatologically acceptable, adhesive polymer. The cleansing is accomplished by applying the formulation to skin as an applique, drying the applique, contacting the dried applique with an adhesive tape type material which bonds with the applique, and then pulling the applique/tape away from the skin.

### Specific Embodiments

These skin cleansers are based on certain naturally occurring and synthetic polymers which are soluble in simple alcohols, or simple alcohol and water mixtures. It has been found that the selected polymers remove surface and follicular debris when formulated and applied without additives which modify their native configuration. Adding polymerizing agents or cross-linkers to the selected polymers reduces the capacity of these appliques to effectively remove soil. Thus polymers are pre-formed, that is they are not presented as monomers and cross-linked when applied to the skin. They may be polymerized just prior to use by mixing monomers and cross-linking agents from separate containers, then after polymerization has occurred, applying the resulting polymeric solution to the skin. But polymers are not formed *in situ*, that is when applied to the skin. These polymers will be dermatologically acceptable within the ambit of their use as skin cleansers.

Polymers include those which have intrinsic adhesive properties and are soluble in simple alcohols or water/simple alcohol mixtures. Useful polymers include PVP, PVP/vinyl acetate, alkylvinyl ethers, alkylvinyl ether/maleic acids and acid salts, or carboxymethyl celluloses. The most preferred polymers are PVPs and methylvinyl ether/maleic acid and acid salts sold by International Speciality Polymers of Wayne, New Jersey, USA. A single polymer, or mixtures, can be used in these formulations.

Forming appliques is a matter of providing a liquid or gel which can be conveniently spread on the skin, which will then quickly dry to an elastic, pliable solid, and which can then be peeled from the skin by some means. The elastic, pliable characteristics are achieved by selecting polymers and polymer mixtures which provide these characteristics. In addition, they should be soluble in solvent with a low vapor pressure so the solvent quickly evaporates when the solution is applied to the skin. Simple alcohols and alcohol/water mixtures are best for this purpose. A simple alcohol is methanol, ethanol or isopropanol. Ethanol is preferred. Methanol may not be useful in certain applications because of its toxicity potential. Pure alcohol may be used as the solvent, though combining water with alcohols is also useful. The solvent may contain up to about 20% water. A preferred alcohol/water mix is one in the range of 90% alcohol/10% water. Another preferred solvent is 95% ethanol/5% water.

Formulations of polymer and solvent will have some degree of viscosity so that when one goes to apply them to the skin they will not flow excessively. It is most desirable to provide an out-of-the-bottle formulation which forms up in the manner of a gel or paste which can be readily spread with the fingers or a pliable device. While it is expected that the polymers at the concentrations used to make these cleansers will impart such qualities, an agent to thicken may be added to a formulation which inherently does not have this property.

Polymer concentrations will range from about 0.1 to 20 percent by weight (weight/volume). Within this range the concentration can be infinitively varied. A more preferred range is between about 2 - 10%, and most formulations will contain about 5% polymer.

Solutions are readily prepared by adding a selected amount of polymer to solvent with stirring or mixing means. This can be done at room temperature, or at slightly elevated temperatures if desired.

To clean skin, a dollop of liquid or gel is placed on the skin and spread into an applique by hand or a soft pliable device. For example, a small amount of material is dispensed onto the fingers and spread over the cheek of the face. Body heat, air flow and ambient heat will evaporate the solvent leaving behind an elastic, pliable essentially dry applique. This should take several minutes, e.g., 3 - 10, after which the applique can be removed. This is accomplished by pulling it off; simply grasp the edge of the applique with the fingers and steadily pull it from the face. An alternative, and preferred method is to take a piece of adhesive tape, or similar material, and touch it to the dried applique. Both are then pulled from the skin with steady, gentle pressure.

Adhesive tape refers to any cloth or plastic patch or strip which has an adhesive coating which sticks to the applique well enough to pull it from the skin. Preferred materials are the Scotch™ tapes sold by 3M Company of Minneapolis, Minnesota, USA, medical adhesive tapes and patches like Blenderm™ also manufactured by 3M Company and Dermiclear™ manufactured by Johnson & Johnson. Preferred adhesives are hypoallergenic acrylics.

The following examples are given to illustrate the invention, not to limit its scope. Reference is made to the claims for what is reserved to the inventors hereunder.

### EXAMPLE 1

### Applique Formulation

A high molecular weight PVP (Plasdone K 120, MW ∼3 million) was dissolved in USP ethanol (typically containing 4 to 5 percent water by weight) at the five percent level (weight/volume). This was accomplished by dispensing 5.0 grams of polymer into 100 grams of USP ethanol under high speed stirring (2000 RPM) at room temperature. Polymer was slowly added into the vortex of the ethanol to prevent clumping. Stirring was terminated when dissolution of the polymer was complete.

A portion of this ethanolic solution was applied to the nose with the fingers and allowed to dry completely (5 - 7 minutes). Then a length of Scotch tape was applied to the surface of the dried applique. Tape and adhered polymer were peeled away from the skin. When the tape was examined under a microscope (Power x 100) several layers of stratum corneum and hair follicles encased by horn were observed.

## Claims

1. A peel-off appliqué composition for cleaning or treating skin comprising a lower alcohol or alcohol/water solvent in which is dissolved between 2 to 10% by weight/volume of a polymer consisting essentially of a non-acrylate, pre-polymerized high molecular weight, dermatologically acceptable adhesive polymer.

2. A composition according to claim 1 where the polymer is polyvinylpyrrolidone (PVP), PVP/vinyl acetate, alkylvinyl ethers, alkylvinyl ether/maleic acids and acid salts, or carboxymethyl celluloses.

3. A composition according to claim 2 wherein the polymer is PVP having a molecular weight of about 3 million or methylvinyl ether/maleic anhydride having a molecular weight between 18,000 and 80,000.

4. A composition according to any one of claims 2 or 3 wherein the polymer is PVP/vinyl acetate or polyethylene oxide.

5. A composition according to any one of claims 2 or 3 wherein the PVP has a molecular weight of 3 million and is present at a concentration of 5% in ethanol.

6. A composition according to any one of claims 1 or 2 where the solvent is an alcohol/water mixture where the alcohol is methanol, ethanol or isopropanol.

7. A method for cleansing skin which comprises:
a. applying to the skin a formulation comprising a lower alcohol or alcohol/water solvent in which is dissolved between 0.1 to 20% by weight/volume of a polymer consisting essentially of a non-acrylate, pre-polymerized high molecular weight, dermatologically acceptable, adhesive polymer;
b. allowing the formulation to dry to form an appliqué; and
c. removing the appliqué by contacting it with adhesive tape and then peeling the appliqué and tape from the skin.

8. A method according to claim 7 which results in the removal of comedones.

9. A skin cleansing product comprising a peel-off appliqué composition for cleaning or treating skin comprising a lower alcohol or alcohol/water solvent in which is dissolved between 0.1 to 20% by weight of a polymer consisting essentially of a non-acrylate, pre-polymerized high molecular weight, dermatologically acceptable adhesive polymer; and adhesive tape for removing the appliqué from the skin.

## Patentansprüche

1. Abschälmittelzusammensetzung (peel-off appliqué composition) zur Reinigung oder Behandlung der Haut, welche einen niederen Alkohol oder Alkohol/Wasser als Lösemittel umfaßt, worin zwischen 2 bis 10 Gew./Vol.% eines Polymers gelöst sind, das im wesentlichen aus einem nichtacrylatischen, vorpolymerisierten, dermatologisch annehmbaren adhäsiven Polymer mit hohem Molekulargewicht besteht.

2. Zusammensetzung gemäß Anspruch 1, worin das Polymer Polyvinylpyrrolidon (PVP), PVP/Vinylacetat, Alkylvinylether, Alkylvinylether/Maleinsäuren und -säuresalze oder Carboxymethylcellulosen ist/sind.

3. Zusammensetzung gemäß Anspruch 2, worin das Polymer PVP mit einem Molekulargewicht von ungefähr 3 Millionen oder Methylvinylether/Maleinanhydrid mit einem Molekulargewicht zwischen 18.000 und 80.000 ist.

4. Zusammensetzung gemäß mindestens einem der Ansprüche 2 oder 3, worin das Polymer PVP/Vinylacetat oder Polyethylenoxid ist.

5. Zusammensetzung gemäß mindestens einem der Ansprüche 2 oder 3, worin das PVP ein Molekulargewicht von 3 Millionen hat und in einer Konzentration von 5 % in Ethanol vorhanden ist.

6. Zusammensetzung gemäß mindestens einem der Ansprüche 1 oder 2, worin das Lösemittel eine Alkohol/Wasser-Mischung ist, worin der Alkohol Methanol, Ethanol oder Isopropanol ist.

7. Verfahren zur Reinigung der Haut, welches umfaßt:
a. Auftragen einer Formulierung auf die Haut, die einen niederen Alkohol oder Alkohol/Wasser als Lösemittel umfaßt, worin zwischen 0,1 bis 20 Gew./Vol.% eines Polymers gelöst ist, das im wesentlichen aus einem nichtacrylatischen, vorpolymerisierten, dermatologisch annehmbaren adhäsiven Polymer mit hohem Molekulargewicht besteht;
b. Trocknenlassen der Formulierung, um ein Mittel zu bilden; und
c. Entfernen des Mittels durch Kontaktieren mit einem Klebestreifen und anschließendes Abschälen des Mittels und des Streifens von der Haut.

8. Verfahren gemäß Anspruch 7, welches in der Entfernung von Komidonen resultiert.

9. Hautreinigungsprodukt, das eine Abschälmittelzusammensetzung zur Reinigung oder Behandlung der Haut, welche einen niederen Alkohol oder Alkohol/Wasser als Lösemittel umfaßt, worin zwischen 0,1 bis 20 Gew.% eines Polymers gelöst sind, das im wesentlichen aus einem nichtacrylatischen, vorpolymerisierten, dermatologisch annehmbaren adhäsiven Polymer mit hohem Molekulargewicht besteht, und Klebestreifen zur Entfernung des Mittels von der Haut umfaßt.

## Revendications

1. Composition apte à former une couche pelable pour le nettoyage ou le traitement de la peau, comprenant un alcool intérieur ou un mélange d'alcool et d'eau comme solvant dans lequel on dissout entre 2 et 10 % en poids/volume d'un polymère constitué essentiellement d'un polymère adhésif non acrylique prépolymérisé, de poids moléculaire élevé et acceptable du point de vue dermatologique.

2. Composition selon la revendication 1, dans laquelle le polymère est constitué de polyvinylpyrrolidone (PVP), de PVP/acétate de vinyle, d'éthers d'alkylvinyle, d'éther d'alkylvinyle/acides maléiques et de sels d'acides ou de carboxyméthylcelluloses.

3. Composition selon la revendication 2, dans laquelle le polymère est constitué de PVP ayant un poids moléculaire d'environ 3 millions ou d'éther de méthylvinyle/anhydride maléique ayant un poids moléculaire compris entre 18 000 et 80 000.

4. Composition selon l'une quelconque des revendications 2 et 3, dans laquelle le polymère est constitué de PVP/acétate de vinyle ou d'oxyde de polyéthylène.

5. Composition selon l'une quelconque des revendications 2 et 3, dans laquelle la PVP a un poids moléculaire de 3 millions et est présente à une concentration de 5 % dans de l'éthanol.

6. Composition selon l'une quelconque des revendications 1 et 2, dans laquelle le solvant est un mélange d'alcool/eau dans lequel l'alcool est le méthanol, l'éthanol ou l'isopropanol.

7. Procédé de nettoyage de la peau comprenant les étapes consistant à :
a. appliquer sur la peau une formulation comprenant un alcool inférieur ou un mélange d'alcool/eau comme solvant dans lequel on dissout entre 0,1 et 20 % en poids/volume d'un polymère constitué essentiellement d'un polymère adhésif non acrylique, prépolymérisé, de poids moléculaire élevé et acceptable du point de vue dermatologique ;
b. laisser sécher la formulation pour former une couche ; et
c. retirer la couche en l'amenant au contact d'un ruban adhésif et en décollant ensuite la couche et le ruban de la peau.

8. Procédé selon la revendication 7 qui aboutit à l'élimination des comédons.

9. Produit de nettoyage de la peau, comprenant une composition apte à former une couche pelable pour le nettoyage ou le traitement de la peau, comprenant un alcool inférieur ou un mélange d'alcool et d'eau comme solvant dans lequel on dissout entre 0,1 et 20 % en poids d'un polymère constitué essentiellement d'un polymère adhésif non acrylique prépolymérisé, de poids moléculaire élevé et acceptable du point de vue dermatologique, et un ruban adhésif pour retirer ladite couche de la peau.
